# FASCICULE DE BREVET EUROPEEN

(11) **EP 3 752 173 B1**
(45) Date de publication et mention de la délivrance du brevet: **20.12.2023**
(21) Numéro de dépôt: 19710055.5
(22) Date de dépôt: 15.02.2019
(51) Int. Cl.: A61K 36/41, A61K 36/481, A61K 31/7028, A61K 31/7034, A61K 31/7048, A61K 31/715, A61K 45/06, A61P 25/28

(54) **COMBINAISON DE RHODIOLE ET D'ASTRAGALE POUR LE TRAITEMENT DE MALADIES NEURODEGENERATIVES**
KOMBINATION VON RHODIOLA UND ASTRAGALUS ZUR BEHANDLUNG VON NEURODEGENERATIVER ERKRANKUNGEN
COMBINATION OF RHODIOLA AND ASTRAGALUS FOR THE TREATMENT OF NEURODEGENERATIVE DISEASES

(30) Priorité: 15.02.2018 FR 1851298; 16.02.2018 FR 1851319
(43) Date de publication de la demande: 23.12.2020
(73) Titulaire: Human by Nature, 31071 Toulouse Cedex 7 (FR)
(72) Inventeur: GOUDET, Gérald, 81500 FIAC (FR)
(74) Mandataire: Santarelli
(86) Numéro de dépôt international: PCT/FR2019/050342
(87) Numéro de publication internationale: WO 2019/158870

(56) Documents cités:
- CN-A- 1 981 857
- KR-A- 20030 007 105
- US-A1- 2008 081 046
- US-A1- 2008 118 583
- "Composition d'ADN Téloméractives ©", HBN , 2 mai 2017 (2017-05-02), XP002784849, Extrait de l'Internet: URL:https://www.adn-astragale.fr/astragale / [extrait le 2018-09-18]
- NABAVI SEYED FAZEL ET AL: "Rhodiola rosea L. and Alzheimer's Disease: From Farm to Pharmacy.", PHYTOTHERAPY RESEARCH : PTR APR 2016, vol. 30, no. 4, avril 2016 (2016-04), pages 532-539, XP002784850, ISSN: 1099-1573
- XIA LEI ET AL: "Neuroprotective effects of astragaloside IV on Parkinson disease models of mice and primary astrocytes", EXPERIMENTAL AND THERAPEUTIC MEDICINE, vol. 14, no. 6, décembre 2017 (2017-12), pages 5569-5575, XP002784851,

## Description

La présente invention se rapporte à l'utilisation d'un extrait de Rhodiole et d'un extrait d'Astragale pour traiter des maladies neurodégénératives, et plus particulièrement des maladies telles qu'Alzheimer ou Parkinson.

Par « maladies dégénératives », on entend l'ensemble des pathologies qui évoluent progressivement vers un développement des déficiences et des atteintes sur le patient. Généralement d'origine génétique, les maladies dégénératives peuvent également être causées par une exposition massive à des substances biologiques et toxiques.

Par « maladie neurodégénérative », on entend une maladie qui affecte, de manière progressive, le fonctionnement du système nerveux. Généralement, le fonctionnement des cellules nerveuses, et plus particulièrement des neurones, se trouve altéré. D'évolution plus ou moins rapide, celles-ci sont fréquemment irréversibles.

Plus particulièrement, on vise par maladies neurodégénératives, les maladies suivantes : la maladie d'Alzheimer, la maladie de Parkinson, la maladie de Huntington, la sclérose latérale amyotrophique, la démence à corps de Lewy, la maladie de Pick, la paralysie supranucléaire progressive, les scléroses en plaques, les maladies à prions humaines ou animales telles que par exemple, l'encéphalopathie spongiforme bovine, et la myopathie.

Du fait de l'allongement de l'espérance de vie, la pose de diagnostic de maladies neurodégénératives telles que la maladie d'Alzheimer ou de Parkinson, est en constante augmentation.

Rare avant 65 ans, la maladie d'Alzheimer est une maladie qui se caractérise par une dégénérescence progressive des neurones, conduisant peu à peu à la perte des fonctions intellectuelles : il s'agit notamment dans un premier temps, de troubles de la mémoire, de troubles des fonctions exécutives et de problèmes d'orientation dans le temps et l'espace. L'évolution progressive de la maladie chez le patient conduit à l'installation irréversible d'une démence.

Rare avant 45 ans, la maladie de Parkinson touche environ 1% des personnes de plus de 65 ans. Cette maladie, d'évolution tout aussi progressive et irréversible que la maladie d'Alzheimer, se caractérise par la destruction d'une population particulière de neurones. Elle provoque des symptômes dits « moteurs » tels qu'une lenteur dans la mise en oeuvre et la coordination des mouvements, une rigidité excessive des muscles et des tremblements et des symptômes « non moteurs » tels que des troubles cognitifs, du sommeil, de perte d'équilibre et/ou une dépression.

Aucun des médicaments prescrits aujourd'hui pour ces deux maladies ne permet de les guérir ou de stopper leur évolution. Ils visent principalement à retarder l'évolution des maladies et tentent d'en traiter les symptômes.

Le travail des inventeurs a permis de mettre en évidence qu'une nouvelle combinaison d'actif permettait de traiter des maladies neurodégénératives, notamment des maladies telles qu'Alzheimer ou Parkinson.

L'invention concerne donc un produit contenant un extrait de Rhodiole et un extrait d'Astragale comme produits de combinaison pour une utilisation simultanée, séparée ou étalée dans le temps dans le traitement d'une maladie neurodégénérative, ledit extrait de Rhodiole étant un extrait de *Rhodiola rosea* et ledit extrait d'Astragale étant un extrait *d'Astragalus membranaceus,* dans lequel l'extrait de *Rhodiola rosea* comporte au moins 1,5% en poids de rosavine sur le poids total d'extrait sec de *Rhodiolia rosea.*

La présente demande divulgue également la combinaison d'un extrait de Rhodiole et d'un extrait d'Astragale pour une utilisation dans le traitement d'une maladie neurodégénérative.

Par « un extrait de Rhodiole », on entend un extrait ou un mélange d'extraits de Rhodiole.

Par « un extrait d'Astragale », on entend un extrait ou un mélange d'extraits d'Astragale.

Comme cela est démontré à l'Exemple 2, la combinaison d'un extrait de Rhodiole et d'un extrait d'Astragale présente un effet curatif synergique dans le cadre d'un modèle de maladie neurodégénérative.

Par ailleurs, comme cela est démontré à l'Exemple 3, la combinaison d'un extrait de Rhodiole et d'un extrait d'Astragale présente également un effet protecteur préventif dans le cadre du même modèle de maladie neurodégénérative.

Plus particulièrement, la maladie neurodégénérative est choisie parmi le groupe constitué par la maladie d'Alzheimer, la maladie de Parkinson, la maladie de Huntington, la sclérose latérale amyotrophique, les maladies à prions telles que par exemple, l'encéphalopathie spongiforme bovine, la démence à corps de Lewy, la maladie de Pick et la myopathie.

Préférentiellement, il s'agit d'une maladie choisie parmi le groupe constitué par la maladie d'Alzheimer, la maladie de Parkinson, la maladie de Huntington, la sclérose latérale amyotrophique, l'encéphalopathie spongiforme bovine et la myopathie et plus préférentiellement, la maladie d'Alzheimer, la maladie de Parkinson, la maladie de Huntington et la myopathie.

Plus particulièrement, il s'agit d'une maladie choisie parmi le groupe constitué par la maladie d'Alzheimer et la maladie de Parkinson.

En effet, les inventeurs ont montré qu'une combinaison d'extraits de Rhodiole et d'Astragale permettait non seulement de maintenir la longueur des neurites dans un modèle de maladie neurodégénérative, en particulier d'Alzheimer, mais également d'obtenir leur allongement. Ce résultat est surprenant puisqu'un extrait de Rhodiole ou d'Astragale seul ne permet pas d'améliorer la longueur des neurites dans un modèle de maladie neurodégénérative voire même, la détériore.

L'utilisation du produit peut être simultanée, séparée ou étalée dans le temps.

A titre d'exemple, le produit peut être administré via une unique prise (comprimé, gélule, etc.) comportant un extrait de Rhodiole et un extrait d'Astragale ou via deux prises distinctes, une première prise comportant un extrait de Rhodiole et une seconde prise comportant un extrait d'Astragale. Dans ce deuxième cas, les deux prises peuvent être effectuées simultanément, séparément ou étalées dans le temps.

Avantageusement, les produits de combinaison, à savoir l'extrait de Rhodiole et l'extrait d'Astragale, sont utilisés de manière simultanée.

Le produit contenant un extrait de Rhodiole et un extrait d'Astragale comme produits de combinaison peut être utilisé pour un traitement préventif et/ou curatif.

Comme cela est démontré dans les Exemples 2 et 3, les produits de combinaison selon l'invention ont démontré un effet préventif et curatif dans le cadre d'un modèle de maladie neurodégénérative et plus particulièrement dans le cadre d'un modèle de la maladie d'Alzheimer.

De préférence, le produit contenant un extrait de Rhodiole et un extrait d'Astragale comme produits de combinaison est utilisé pour un traitement curatif de neurones, notamment dans le cadre de la maladie d'Alzheimer.

Plus particulièrement, le traitement curatif de neurones s'effectue notamment par allongement des neurites desdits neurones.

Avantageusement, dans le produit contenant un extrait de Rhodiole et un extrait d'Astragale comme produits de combinaison, le ratio en poids sec de l'extrait d'Astragale sur l'extrait de Rhodiole est compris entre 0,5 et 1,5.

On notera que dans le cadre de la présente demande, et sauf stipulation contraire, les gammes de valeurs indiquées s'entendent bornes incluses.

Préférentiellement, le ratio en poids sec de l'extrait d'Astragale sur l'extrait de Rhodiole est compris entre 0,8 et 1,2, préférentiellement, de l'ordre de 1.

L'extrait de Rhodiole est un extrait de *Rhodiola rosea.*

De préférence, le produit contenant un extrait de Rhodiole et un extrait d'Astragale comme produits de combinaison comporte une dose d'au moins 50 mg/jour d'un extrait de Rhodiole, ledit extrait étant un extrait de *Rhodiola rosea.*

Plus particulièrement, pour un humain adulte, la dose d'extrait de *Rhodiola rosea* peut être comprise entre 50 et 500mg/jour, plus préférentiellement, entre 100 et 250mg/jour, encore plus préférentiellement de l'ordre de 150mg/jour.

Plus généralement, pour un animal, le produit contenant un extrait de Rhodiole et un extrait d'Astragale comme produits de combinaison peut comporter une dose d'extrait sec de *Rhodiola rosea* d'au moins 0,5mg/kg/jour, préférentiellement une dose comprise entre 1 et 10mg/kg/jour, par exemple de l'ordre de 1,5 à 2mg/kg/jour.

De préférence, ces doses sont des doses d'extraits secs.

Préférentiellement, l'extraction est effectuée à partir de poudre de racines *Rhodiola rosea.*

La *Rhodiola rosea,* également appelée racine d'or ou racine arctique, est une plante vivace de la famille des crassulacées, qui pousse essentiellement dans les régions froides du monde, tels les régions froides de l'Asie, de la Sibérie, de l'Amérique du Nord et les régions montagneuses d'Europe (Alpes, Pyrénées, Carpates, Scandinavie, Islande, grande Bretagne et Irlande). Cette plante est utilisée, notamment en médecine traditionnelle chinoise, pour combattre le stress et les troubles de l'humeur. Avantageusement, *Rhodiola rosea* provient d'Asie, de préférence de Chine.

Avantageusement, l'extrait de racines de *Rhodiola rosea* est un extrait hydroalcoolique. Typiquement, le solvant d'extraction de l'extrait est un mélange eau-éthanol, tel qu'un mélange eau-éthanol comportant au moins 50% en volume, préférentiellement au moins 60% en volume, tel que par exemple 70% en volume d'eau.

Avantageusement, le ratio en poids mélange eau-éthanol : poudre de racines *Rhodiola rosea* est compris entre 16 :1 et 4 :1, préférentiellement entre 12 :1 et 6 :1, tel que de l'ordre de 10 :1 à 8 :1.

L'extraction de la Rhodiole peut être conduite selon les méthodes usuelles (percolation, infusion, décoction, macération) bien connues de l'homme de l'art. De préférence, l'extraction de la poudre est répétée plusieurs fois. Avantageusement, l'extraction est conduite à chaud, de préférence à une température modérée (40 à 100°C). Après extraction, l'extrait est de préférence séché.

L'extrait de *Rhodiola rosea* comporte au moins 1,5% en poids de rosavine sur le poids total d'extrait sec de *Rhodiolia rosea.*

Par rosavine (ou « rosavin » en anglais), on désigne un glycoside d'alcool cinnamique, extrait de la Rhodiole, et plus particulièrement de la *Rhodiola rosea.*

Préférentiellement, l'extrait de *Rhodiola rosea* comporte au moins 2% en poids de rosavine, plus préférentiellement, au moins 2,5% en poids de rosavine, encore plus préférentiellement, au moins 3% en poids de rosavine, sur le poids total d'extrait sec de *Rhodiola rosea.*

Avantageusement, l'extrait de *Rhodiola rosea* comporte également au moins 0,25% en poids de salidroside, sur le poids total d'extrait sec de *Rhodiola rosea.*

Par salidroside (également appelé « rhodioloside »), on désigne un glucoside phénylpropanoïde, extrait de la Rhodiole, et plus particulièrement de la *Rhodiola rosea.*

Préférentiellement, l'extrait de *Rhodiola rosea* comporte au moins 0,5% en poids de salidroside, plus préférentiellement, au moins 0,75% en poids de salidroside, encore plus préférentiellement, au moins 1% en poids de salidroside, sur le poids total d'extrait sec de *Rhodiola rosea.*

Plus particulièrement, l'extrait de *Rhodiola rosea* comporte au moins 1,5% en poids de rosavine et au moins 0,25% en poids de salidroside, préférentiellement, au moins 2% en poids de rosavine et au moins 0,5% en poids de salidroside, plus préférentiellement, au moins 2,5% en poids de rosavine et au moins 0,75% en poids de salidroside, encore plus préférentiellement, au moins 3% en poids de rosavine et au moins 1% en poids de salidroside, sur le poids total d'extrait sec de *Rhodiolia rosea.*

L'extrait de Rhodiole, et plus particulièrement l'extrait sec de *Rhodiola rosea,* peut comporter également avantageusement de la rosarine et/ou de la rosine.

Comme indiqué ci-avant, l'extrait de Rhodiole est utilisé en combinaison avec un extrait d'Astragale.

L'extrait d'Astragale est un extrait *d'Astragalus membranaceus.*

De préférence, le produit contenant un extrait de Rhodiole et un extrait d'Astragale comme produits de combinaison comporte au moins une dose de 50 mg/jour d'un extrait d'Astragale, ledit extrait étant un extrait *d'Astragalus membranaceus.*

Plus particulièrement, pour un humain adulte, la dose d'extrait *d'Astragalus membranaceus* peut être comprise entre 50 et 500mg/jour, plus préférentiellement, entre 100 et 250mg/jour, encore plus préférentiellement de l'ordre de 150mg/jour.

Plus généralement, pour un animal, le produit contenant un extrait de Rhodiole et un extrait d'Astragale comme produits de combinaison peut comporter une dose d'extrait sec *d'Astragalus membranaceus* d'au moins 0,5mg/kg/jour, préférentiellement une dose comprise entre 1 et 3mg/kg/jour, par exemple de l'ordre de 1,5 à 2mg/kg/jour.

De préférence, ces doses sont des doses d'extraits secs.

Préférentiellement, l'extraction est effectuée à partir de poudre de racines *d'Astragalus membranaceus.*

*L'Astragalus membranaceus*, également appelée *Astragalus penduliflorus* ou *Astragalus propinquus*, est une plante de la famille des fabacées qui pousse en Asie, plus particulièrement au nord de la Chine et dans les provinces du Yunnan et du Sichuan. Cette plante est utilisée, notamment en médecine traditionnelle chinoise, pour stimuler le système immunitaire et combattre les infections. Avantageusement, *l'Astragalus membranaceus* provient d'Asie, de préférence de Chine.

Avantageusement, l'extrait *d'Astragalus membranaceus* comporte au moins 0,2% en poids d'astragalosides sur le poids total d'extrait sec *d'Astragalus membranaceus.*

Par astragalosides (parfois désigné « astrangaloside »), on désigne des oligoglycosides triterpéniques, extraits de l'Astragale, et plus particulièrement de *l'Astragalus membranaceus.*

Préférentiellement, l'extrait *d'Astragalus membranaceus* comporte au moins 0,4% en poids d'astragalosides, plus préférentiellement, au moins 0,6% en poids d'astragalosides, encore plus préférentiellement, au moins 0,8% en poids d'astragalosides, sur le poids total d'extrait sec *d'Astragalus membranaceus.*

Avantageusement, l'extrait comporte un mélange d'astragalosides, c'est-à-dire au moins deux astragalosides choisis parmi les astragalosides I, II, III, IV, V, VI et VII.

De préférence, l'extrait comporte un mélange des 7 astragalosides précités.

Avantageusement, l'extrait *d'Astragalus membranaceus* comporte au moins 10% en poids de polysaccharides, sur le poids total d'extrait sec *d'Astragalus membranaceus.*

Préférentiellement, l'extrait *d'Astragalus membranaceus* comporte au moins 12% en poids de polysaccharides, plus préférentiellement, au moins 14% en poids, encore plus préférentiellement, au moins 16% en poids de polysaccharides sur le poids total d'extrait sec *d'Astragalus membranaceus.*

Plus particulièrement, l'extrait *d'Astragalus membranaceus* comporte au moins 0,2% en poids d'astragalosides et au moins 10% en poids de polysaccharides, préférentiellement, au moins 0,4% en poids d'astragalosides et au moins 12% en poids de polysaccharides, plus préférentiellement, au moins 0,6% en poids d'astragalosides et au moins 14% en poids de polysaccharides, encore plus préférentiellement, au moins 0,8% en poids d'astragalosides et au moins 16% en poids de polysaccharides, sur le poids total d'extrait sec *d'Astragalus membranaceus.*

Avantageusement, l'extrait *d'Astragalus membranaceus* est préparé à partir de deux extraits de racines *d'Astragalus membranaceus,* un premier extrait, hydroalcoolique, et un second extrait, aqueux.

Typiquement, le solvant d'extraction du premier extrait est un mélange eau-éthanol. Préférentiellement, le mélange eau-éthanol comporte au moins 50% en volume, préférentiellement au moins 75% en volume, tel que par exemple 85% en volume d'éthanol.

Avantageusement, le ratio en poids mélange eau-éthanol : poudre de racines *d'Astragalus membranaceus* est compris entre 200 :1 et 50 :1, préférentiellement entre 150 :1 et 100 :1, tel que de l'ordre de 125 :1.

L'extraction du second extrait est effectuée par un solvant constitué d'eau.

Typiquement, le ratio en poids eau : poudre de racines *d'Astragalus membranaceus* est compris entre 10 :1 et 2 :1, préférentiellement entre 7 :1 et 3 :1, tel que de l'ordre de 5 :1.

L'extraction de l'Astragale peut également être conduite selon les méthodes usuelles (percolation, infusion, décoction, macération) bien connues de l'homme de l'art. De préférence, l'extraction de la poudre est répétée plusieurs fois. Avantageusement, l'extraction est conduite à chaud, de préférence à une température modérée (40 à 100°C). Après extraction, l'extrait est de préférence séché.

Selon un mode de réalisation particulier de l'invention, le produit contenant un extrait de Rhodiole et un extrait d'Astragale comme produits de combinaison comporte en outre un ingrédient choisi parmi le groupe constitué par le tocophérol, le sélénium, le silicium, le zinc, le magnésium, les polyphénols autres que ceux issus des extraits de Rhodiole et d'Astragale, ou leurs mélanges.

De préférence, le tocophérol est introduit dans le produit à une quantité comprise entre 0,05% et 2% en poids sur le poids total de produit.

De préférence, le sélénium est introduit dans le produit à une quantité comprise entre 0,0001% et 0,005% en poids sur le poids total de produit, par exemple sous la forme de levure de sélénium.

De préférence, le silicium est introduit dans le produit à une quantité comprise entre 6% et 10% en poids sur le poids total de produit, par exemple sous la forme de diatomées naturelles (non calcinées), de dioxyde de silicium, de silicate(s) et/ou d'acide orthosilicique (noms génériques : silicium organique, silicium biogénique).

De préférence, le zinc est introduit dans le produit à une quantité comprise entre 0,01% et 0,5% en poids sur le poids total de produit, par exemple sous la forme de sel(s) de zinc, tel que du citrate, du sulfate et/ou du bisglycinate de zinc.

De préférence, le magnésium est introduit dans le produit à une quantité comprise entre 5% et 50% en poids sur le poids total de produit, par exemple sous la forme de sel(s) de magnésium tels que du glycérophosphate de magnésium et/ou du citrate de magnésium.

Les polyphénols peuvent être présents dans le produit par l'introduction d'un extrait végétal, tel qu'un extrait de Vitis vinifera (vigne rouge) et/ou de Polygonum cuspidatum (Renouée du Japon). De préférence, les polyphénols sont introduits dans le produit à une quantité comprise entre 1% et 10% en poids sur le poids total de produit.

De préférence, un polyphénol est du resvératrol (naturel ou synthétique). Avantageusement, le resvératrol est introduit dans le produit à une quantité comprise entre 0,1% et 3% en poids sur le poids total de produit.

Dans un mode de réalisation préféré de l'invention, le produit contenant un extrait de Rhodiole et un extrait d'Astragale comme produits de combinaison comporte l'ensemble des ingrédients ci-avant.

Dans ce mode de réalisation préféré, le produit contenant un extrait de Rhodiole et un extrait d'Astragale comme produits de combinaison peut alors comporter au moins 3,5% en poids d'un extrait de Rhodiole, préférentiellement, au moins 4% en poids, plus préférentiellement, au moins 4,5% en poids d'un extrait de Rhodiole, sur le poids total de produit.

Il peut également comporter au moins 3,5% en poids d'un extrait d'Astragale, préférentiellement au moins 4% en poids, plus préférentiellement, au moins 4,5% en poids d'un extrait d'Astragale sur le poids total de produit.

De préférence, les extraits sont des extraits secs.

Avantageusement, les extraits de Rhodiole et d'Astragale sont tels que décrits ci-avant.

Selon un mode de réalisation particulièrement préféré, le produit selon l'invention contient :
- un extrait de *Rhodiola rosea* comportant au moins 1,5% en poids de rosavine et au moins 0,25% en poids de salidroside, préférentiellement, au moins 2% en poids de rosavine et au moins 0,5% en poids de salidroside, plus préférentiellement, au moins 2,5% en poids de rosavine et au moins 0,75% en poids de salidroside, encore plus préférentiellement, au moins 3% en poids de rosavine et au moins 1% en poids de salidroside, sur le poids total d'extrait sec de *Rhodiolia rosea,* et
- un extrait *d'Astragalus membranaceus* comportant au moins 0,2% en poids d'astragalosides et au moins 10% en poids de polysaccharides, préférentiellement, au moins 0,4% en poids d'astragalosides et au moins 12% en poids de polysaccharides, plus préférentiellement, au moins 0,6% en poids d'astragalosides et au moins 14% en poids de polysaccharides, encore plus préférentiellement, au moins 0,8% en poids d'astragalosides et au moins 16% en poids de polysaccharides, sur le poids total d'extrait sec *d'Astragalus membranaceus.*

Avantageusement, selon ce mode de réalisation particulièrement préféré, le produit contenant les extraits de *Rhodiolia rosea* et *d'Astragalus membranaceus,* ainsi que lesdits extraits, sont tels que décrits ci-avant, y inclus les modes de réalisation et notamment, les ratios, doses et pourcentages en poids décrits ci-avant.

La présente demande divulgue également une méthode de traitement d'une maladie neurodégénérative comportant l'administration simultanée, séparée ou étalée dans le temps d'un extrait de Rhodiole et d'un extrait d'Astragale. Elle divulgue également une méthode de prévention d'une maladie neurodégénérative comportant l'administration simultanée, séparée ou étalée dans le temps d'un extrait de Rhodiole et d'un extrait d'Astragale.

Avantageusement, l'extrait de Rhodiole et l'extrait d'Astragale forment un produit tel qu'un produit de combinaison, administrable au patient sous forme de gélule(s), pilule(s) et/ou comprimé(s).

Les caractéristiques particulières, avantageuses et préférées des méthodes selon l'invention sont telles que décrites ci-avant, notamment en ce qui concerne les extraits, le produit et les doses.

D'autres caractéristiques et avantages de l'invention, apparaîtront dans les exemples qui suivent, donnés à titre illustratif, avec référence aux Figures :
- La Figure 1 présente quatre diagrammes illustrant les résultats de cytotoxicité de l'extrait de Rhodiole, d'Astragale, de la combinaison d'extraits (« Mix ») et du produit "ADN-Téloméractives", après 48 heures d'exposition. Les résultats sont exprimés en un écart-type moyen +/- de 3 réplicats ;
- La Figure 2 présente quatre diagrammes illustrant l'effet curatif de l'extrait de Rhodiole, d'Astragale, de la combinaison d'extraits et du produit "ADN-Téloméractives" sur la longueur des neurites après 48 heures dans le modèle Alzheimer. Les résultats sont exprimés en un écart-type moyen +/- de 3 réplicats ;
- La Figure 3 comporte trois photographies illustrant l'effet de 250 µg/mL de Rhodiole sur des (cellules) SH-SY5Y différenciées préalablement exposées à 10 µM d'Aftin-5 pendant 48 heures. Grossissement x20, noyaux en bleu, tubuline B3 en vert ;
- La Figure 4 comporte trois photographies illustrant l'effet de 250 µg/mL d'Astragale sur des (cellules) SH-SY5Y différenciées préalablement exposées à 10 µM d'Aftin-5 pendant 48 heures. Grossissement x20, noyaux en bleu, tubuline B3 en vert ;
- La Figure 5 comporte trois photographies illustrant l'effet de 250 µg/mL de la combinaison d'extraits de Rhodiole et d'Astragale, sur des (cellules) SH-SY5Y différenciées préalablement exposées à 10 µM d'Aftin-5 pendant 48 heures. Grossissement x20, noyaux en bleu, tubuline B3 en vert ;
- La Figure 6 comporte trois photographies illustrant l'effet de 25,00 µg/mL de produit "ADN-Téloméractives" sur des (cellules) SH-SY5Y différenciées préalablement exposées à 10 µM d'Aftin-5 pendant 48 heures. Grossissement x20, noyaux en bleu, tubuline B3 en vert ;
- La Figure 7 est un diagramme illustrant l'effet du produit "ADN Téloméractives" sur la réparation de lésions de l'ADN (8oxoG, Etheno, Glycols, AbaS, CPD-64) dans des cellules HepG2 ;
- La Figure 8 est un diagramme illustrant l'effet du génotoxique EMS sur la réparation de lésions de l'ADN (8oxoG, Etheno, Glycols, AbaS, CPD-64) dans des cellules HepG2 ;
- La Figure 9 est un diagramme illustrant l'effet d'un prétraitement et d'un post-traitement par le produit "ADN Téloméractives" sur la réparation de lésions de l'ADN (8oxoG, Etheno, Glycols, AbaS, CPD-64) induite par EMS dans des cellules HepG2 ;
- La Figure 10 présente trois diagrammes illustrant l'effet d'une combinaison d'extraits de Rhodiole et d'Astragale ne comprenant pas d'astragalosides (Combinaison 1), d'une combinaison d'extraits de Rhodiole et d'Astragale ne comprenant pas de polysaccharides (Combinaison 2) et de la combinaison d'extraits de Rhodiole et d'Astragale décrite à l'Exemple 1 (Combinaison 4) sur la longueur des neurites après 48 heures dans le modèle Alzheimer ; et
- La Figure 11 présente deux diagrammes illustrant l'effet d'une combinaison d'extraits de Rhodiole et d'Astragale ne comprenant pas de rosavine (Combinaison 3) et de la combinaison d'extraits de Rhodiole et d'Astragale décrite à l'Exemple 1 (Combinaison 4) sur la longueur des neurites après 48 heures dans le modèle Alzheimer.

### EXEMPLE 1 : Préparation de produits pour une utilisation selon l'invention

### 1. Matériel et méthodes

### 1.1 Produits

Les produits suivants ont été utilisés :
- Un extrait de Rhodiole obtenu par extraction hydroalcoolique de racines broyées de *Rhodiola rosea,* le solvant d'extraction étant constitué de 70% en volume d'eau et 30% en volume d'éthanol, dans un ratio en poids solvant : poudre de racines de l'ordre de 8 :1. Cet extrait de Rhodiole comporte au moins 3% en poids de rosavine et au moins 1% en poids de salidroside, sur le poids total d'extrait sec de Rhodiole ;
- Un premier extrait d'Astragale obtenu par extraction hydroalcoolique de racines broyées *d'Astragalus membranaceus,* le solvant d'extraction étant constitué de 15% en volume d'eau et 85% en volume d'éthanol, dans un ratio en poids solvant : poudre de racines de l'ordre de 125 :1 ;
- Un second extrait d'Astragale obtenu par extraction à l'eau pure de racines broyées *d'Astragalus membranaceus,* dans un ratio en poids eau : poudre de racines de l'ordre de 5 :1 ;
- Le produit « ADN Téloméractives » commercialisé par HBN, comportant la combinaison d'un extrait de Rhodiole et d'un extrait d'Astragale. Le produit comporte également du tocophérol, du sélénium sous forme de levures, du silicium notamment sous forme de diatomées marines, du zinc, du magnésium, un extrait de *Vitis vinifera* ainsi qu'un extrait *de Polygonum cuspidatum* comportant 25% de resvératrol. Le produit comporte également diverses vitamines.

### Préparation de l'extrait combiné d'Astragale :

Les deux extraits d'Astragale mentionnés ci-avant sont mélangés de manière à obtenir un extrait combiné d'Astragale comportant au moins 16% en poids de polysaccharides et au moins 0,8% en poids d'un mélange d'astragalosides I, II, III, IV, V, VI et VII, sur le poids total d'extrait sec d'Astragale.

### Préparation de la combinaison d'extraits de Rhodiole et d'Astragale :

La combinaison d'un extrait de Rhodiole et d'un extrait d'Astragale pour une utilisation selon l'invention est préparée par mélange de l'extrait de Rhodiole mentionné ci-avant avec l'extrait combiné d'Astragale dans un ratio en poids de 1 :1.

Des solutions aqueuses de l'extrait de Rhodiole, de l'extrait combiné d'Astragale, de la combinaison d'extraits et du produit « ADN Téloméractives » ont été fraîchement préparées avec une gamme de 8 concentrations finales (0,00125 - 0,0025 - 0,0050 - 0,0125 - 0,025 - 0,05 - 0,125 - 0,25 mg/mL).

### 1.2 Culture cellulaire et différenciation

Des cellules SH-SY5Y (ATCC, CRL-2266) ont été cultivées et repiquées selon les recommandations du fournisseur.

Tous les essais ont été réalisés dans trois réplicats techniques en utilisant des microplaques à 96 puits. Après l'ensemencement, les cellules SH-SY5Y ont été différenciées par exposition à l'acide rétinoïque (10 µM) pendant 4 jours.

### 1.3 Fixation cellulaire et coloration

Après chaque essai, les cellules ont été fixées en utilisant un mélange de méthanol et d'acétone à température ambiante pendant 5 minutes, avant coloration des noyaux avec Hoechst et immunocoloration de la tubuline β3 (anticorps secondaire conjugué à un fluorochrome Alexa 488).

### 1.4 Cytotoxicité

La cytotoxicité a été évaluée en utilisant la coloration et le comptage des noyaux cellulaires (le nombre de cellules a été évalué en utilisant MetaXpress (Molecular Devices).)

### 1.5 Analyse statistique

La comparaison statistique des résultats par rapport aux conditions de contrôle a été réalisée en utilisant un test t. La signification a été exprimée avec le symbole "*" quand p <0,05.

### 2. Résultats sur la cytotoxicité de l'Astragale, du Rhodiole, de la combinaison d'extraits et du produit "ADN-Téloméractives" (ADN-T)

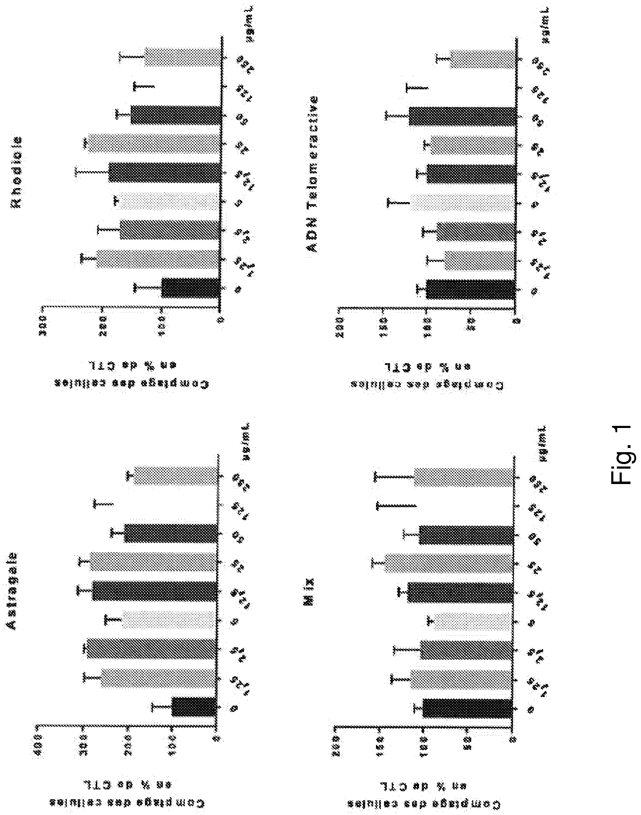

La cytotoxicité a été évaluée sur des (cellules) SH-SY5Y différenciées après 48 heures d'exposition à l'extrait d'Astragale, à l'extrait de Rhodiole, à la combinaison d'extraits et au produit "ADN Téloméractives". Aucun des produits n'a montré d'effet cytotoxique (Voir Figure 1). L'Astragale et le Rhodiole ont induit une augmentation de la viabilité par rapport au contrôle. La viabilité des (cellules) SHSY-5Y exposées à la combinaison d'extraits et au produit "ADN-Téloméractives" est proche de la viabilité du contrôle, quelles que soient les doses testées.

### EXEMPLE 2 : Effet synergique d'un extrait de Rhodiole et d'un extrait d'Astragale pour une utilisation selon l'invention

### 1. Matériel et méthodes

Les produits, la culture cellulaire et différentiation, la fixation cellulaire et coloration, la cytotoxicité et l'analyse statistique sont tels qu'indiqués à l'Exemple 1.

### 1.1 Test curatif

Des modèles de maladies neurodégénératives peuvent être mis en place en laboratoire en utilisant des lignées cellulaires neuronales différenciées. Avec des inducteurs chimiques (Aftin-5, dans le cas présent), un modèle de maladie d'Alzheimer peut être obtenu avec la lignée cellulaire de neuroblastome humain SH-SY5Y. Les propriétés neuroprotectrices et curatives de la combinaison d'extraits sont évaluées à la suite d'une agression neurotoxique, en comptant les neurones et en mesurant la longueur des neurites.

Les cellules ont été exposées pendant 2 jours avec 10 µM d'Aftin-5 seul. Ensuite, elles ont été traitées 48 heures avec les dilutions aqueuses de Rhodiole, d'Astragale, d'une combinaison d'extraits, de produit "ADN-Téloméractives" ou simplement d'eau (contrôle), en co-incubation avec de l'Aftin-5 (10 µM). Après la fixation et l'étiquetage, l'excroissance des neurites et le nombre de cellules ont été évalués en utilisant MetaXpress (Molecular Devices).

### 1.2 Imagerie cellulaire et analyse d'images

Les images ont été obtenues à l'aide d'un microscope automatisé ImageXpress^{®} Micro XLS (Molecular Devices), avec un objectif 20X. Deux filtres dichroïques ont été utilisés simultanément pour détecter spécifiquement les différentes sondes utilisées. Quatre champs par puits ont été enregistrés et analysés individuellement.

Les images ont été analysées en utilisant le logiciel MetaXpress (Molecular Devices).

### 2. Résultats : Effet curatif

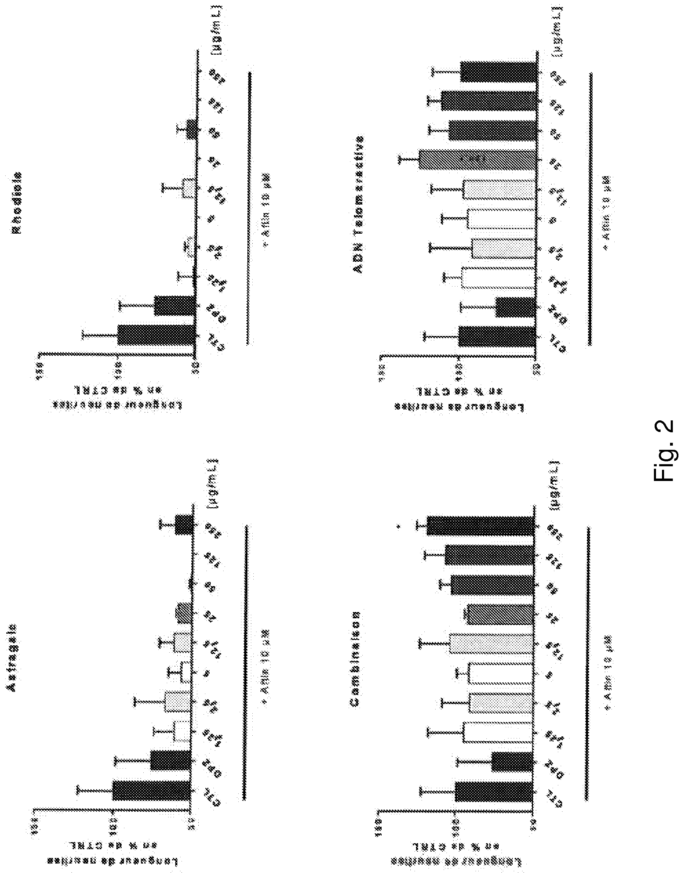

Les résultats sont présentés sur la Figure 2.

Sur cette Figure, on peut constater que la Rhodiole et l'Astragale n'ont pas guéri le phénotype neurodégénératif induit par 48 heures d'exposition à 10 µM d'Aftin-5. Au contraire, à toutes les doses testées, on constate une longueur de neurites inférieure à celle affichée par le contrôle.

En revanche, on peut constater que la combinaison d'extraits de Rhodiole et d'Astragale a montré un effet curatif accru dépendant de la dose contre le phénotype Alzheimer induit. L'effet de la dose est passé de 12,50 µg/mL à 250 µg/mL. À 250 µg/mL, l'effet curatif était significativement différent du contrôle avec une augmentation de 119,1% de la longueur des neurites. Un effet de dose en forme de U a également été observé avec le produit "ADN-Téloméractives" avec un pic à 25,00 µg/mL pour une longueur de neurites à 125,9. Cette valeur n'était pas significativement différente du contrôle, mais comparé à la population cellulaire entière, une différence statistique est montrée par le test t.

Il en résulte que la Rhodiole seule et l'Astragale seule ne permettent pas d'améliorer la longueur des neurites : au contraire, celle-ci est détériorée par rapport au contrôle.

En revanche, la combinaison des extraits de Rhodiole et d'Astragale permet, de manière surprenante un maintien et même un allongement de la longueur des neurites.

Il en résulte que la combinaison d'extraits présente un effet synergique curatif dans un modèle de maladies neurodégénératives et plus particulièrement, un modèle de la maladie d'Alzheimer.

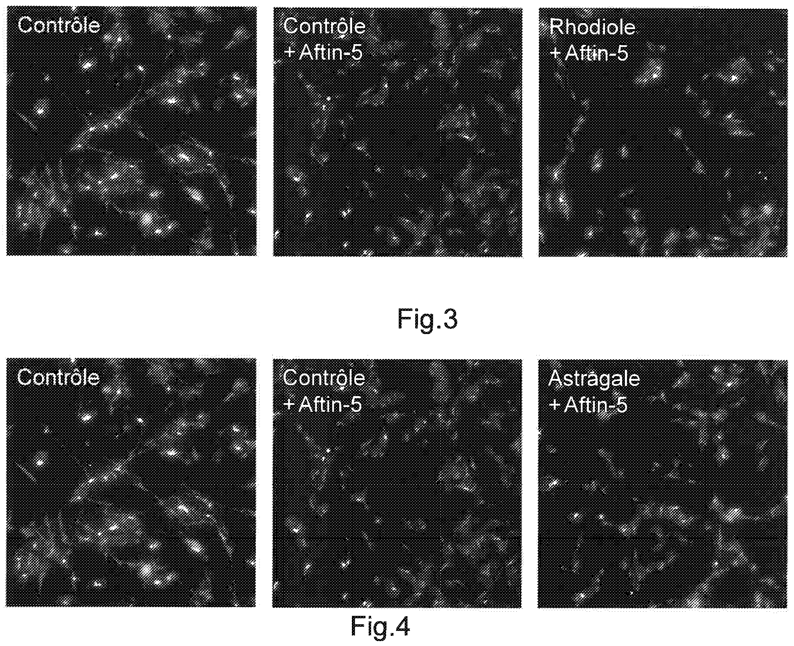

La Figure 3 comporte trois photographies illustrant l'effet de 250 µg/mL de Rhodiole sur des (cellules) SH-SY5Y différenciées préalablement exposées à 10 µM d'Aftin-5 pendant 48 heures. On constate sur cette Figure que, comparées au contrôle, les cellules exposées à l'Aftin-5 montrent une réduction significative de la longueur des neurites. Les cellules co-exposées à l'Aftin-5 et au Rhodiole montrent une longueur des neurites plus petite que celles ayant subie une exposition à l'Aftin-5 seul.

La Figure 4 comporte trois photographies illustrant l'effet de 250 µg/mL d'Astragale sur des (cellules) SH-SY5Y différenciées préalablement exposées à 10 µM d'Aftin-5 pendant 48 heures. Comme sur la Figure précédente, on constate sur cette Figure que, comparées au contrôle, les cellules exposées à l'Aftin-5 montrent une réduction significative de la longueur des neurites. Les cellules co-exposées à l'Aftin-5 et à l'Astragale montrent une longueur des neurites plus petite que celles ayant subie une exposition à l'Aftin-5 seul.

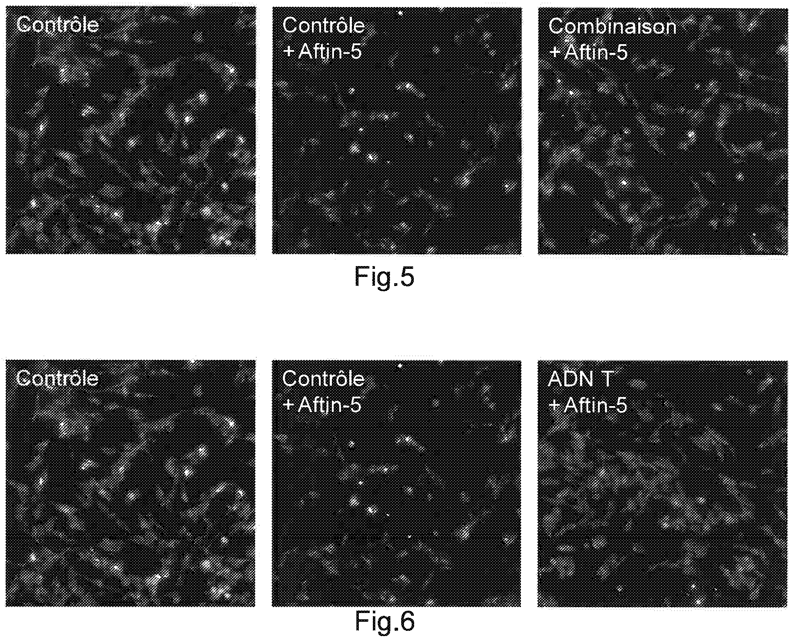

La Figure 5 comporte trois photographies illustrant l'effet de 250 µg/mL de la combinaison d'extraits de Rhodiole et d'Astragale, sur des (cellules) SH-SY5Y différenciées préalablement exposées à 10 µM d'Aftin-5 pendant 48 heures. Comme sur les Figures précédentes, on constate sur cette Figure que, comparées au contrôle, les cellules exposées à l'Aftin-5 montrent une réduction significative de la longueur des neurites. Les cellules co-exposées à l'Aftin-5 et à 250 µg/mL de la combinaison d'extraits de Rhodiole et d'Astragale montrent une meilleure viabilité et longueur des neurites plus importante que celles ayant subie une exposition à l'Aftin-5 seul.

La Figure 6 comporte trois photographies illustrant l'effet de 25,00 µg/mL de produit "ADN-Téloméractives" sur des (cellules) SH-SY5Y différenciées préalablement exposées à 10 µM d'Aftin-5 pendant 48 heures. Comme sur les Figures précédentes, on constate sur cette Figure que, comparées au contrôle, les cellules exposées à l'Aftin-5 montrent une réduction significative de la longueur des neurites. Les cellules co-exposées à l'Aftin-5 et à 25,00 µg/mL de produit "ADN-Téloméractives" montrent une meilleure viabilité et une longueur des neurites plus importante que celles ayant subie une exposition à l'Aftin-5 seul.

### EXEMPLE 3 : Effet neuroprotecteur d'un extrait de Rhodiole et d'un extrait d'Astragale pour une utilisation selon l'invention

### 1. Matériel et méthodes

Les produits, la culture cellulaire et différentiation, la fixation cellulaire et coloration, la cytotoxicité et l'analyse statistique sont tels qu'indiqués à l'Exemple 1.

### 1.1 Test de neuroprotection

Les cellules ont été exposées pendant 48 heures à des dilutions aqueuses de Rhodiole, d'Astragale, d'une combinaison d'extraits et de produit "ADN-Téloméractives", avec 30 µM d'Aftin-5 (inducteur phénotypique de la maladie d'Alzheimer). Après fixation et marquage, l'excroissance des neurites et le nombre de cellules ont été évalués en utilisant MetaXpress (Molecular Devices).

### 2. Résultats : Effet neuroprotecteur

La Rhodiole, l'Astragale, la combinaison d'extrait et le produit "ADN-Téloméractives" ont montré un effet protecteur significatif contre le phénotype de la maladie d'Alzheimer induit par Aftin-5 (30 µM)). L'Astragale et la Rhodiole ont tous deux présenté un effet neuroprotecteur à 1,25 et 2,50 µg/mL, ce qui a augmenté la longueur des neurites respectivement à 129,9 et 127,9. Le mélange d'extraits d'Astragale et de Rhodiole a induit un effet neuroprotecteur à 1,25 µg/mL, avec une longueur de neurite augmentée à 125,6% par rapport au témoin. Le produit "ADN-Téloméractives" induit un effet neuroprotecteur à 2,50 µg/mL, la longueur des neurites étant de 126,8% par rapport au contrôle.

### EXEMPLE 4 : Effet du produit « ADN-Téloméractives » sur les systèmes de réparation de l'ADN

### 1. Matériel et méthodes

### 1.1 Solubilisation du produit « ADN-Téloméractives » (ADN-T)

Après broyage du produit "ADN-Téloméractives", une solution mère aqueuse est préparée à 12,5 mg/mL. Elle est agitée à 37°C pendant 1 heure. Les résidus sont ensuite éliminés par centrifugation 1500 rpm pendant 5 minutes.

### 1.2 Modèle

Le modèle consiste à étudier l'implication des systèmes de réparation de l'ADN dans l'effet protecteur du produit "ADN-Téloméractives" vis-à-vis des génotoxiques, par une approche *in vitro* sur des cellules HepG2, mises en contact avec l'agent génotoxique EMS (méthane sulfonate d'éthyle).

### 1.3 Plan d'expérience

1. Prétraitement des cellules par la solution de produit "ADN-Téloméractives"
   Jour 0 : à 9h00 ensemencement des puits avec les cellules puis à 15h, ajout du produit "ADN-Téloméractives" à 40µg/mL.
2. Introduction du composé génotoxique EMS
   Jour 2 : à 15h00, introduction de l'EMS à deux concentrations : 0,2mM et 1mM.
3. Remise des cellules en présence du produit "ADN-Téloméractives"
   Jour 3 : à 8h00, ajout du "ADN-Téloméractives" à 40µg/mL.
4. Récolte et analyses
   Jour 4 : à 8h00, récolte.

Un contrôle sans produit "ADN-Téloméractives" est réalisé en parallèle.

**Tableau 1 : Listes des conditions testées**

| Prétraitement ADN-T (µg/mL) | Traitement génotoxique (mM) | Post-traitement ADN-T (µg/mL) |
|---|---|---|
| 0 | 0 | 0 |
| 0 | 0,2 | 0 |
| 0 | 1 | 0 |
| 40 | 0 | 40 |
| 40 | 0,2 | 40 |
| 40 | 1 | 40 |

### 1.4 Analyses

Les extraits sont déposés sur une puce fonctionnalisée par des plasmides comportant des séries de lésions de l'ADN spécifiques :
- 8oxoG (8oxoG)
- éthénobases (Etheno)
- Glycols de thymine et cytosine (Glycols)
- sites abasiques (AbaS)
- photoproduits (dimères de pyrimidine et photoproduits 6-4) (CPD-64)

Les enzymes de réparation de l'ADN, contenues dans les extraits, excisent les lésions (ou le fragment d'ADN entourant les lésions) et incorporent un marqueur fluorescent (dCTP-Cy3) lors de la resynthèse d'ADN.

Le signal fluorescent est quantifié à l'aide d'un scanner. Il est proportionnel aux capacités d'Excision/Resynthèse de chaque extrait vis-à-vis de chacune des lésions.

Ce test permet de caractériser les systèmes de Réparation par Excision de Bases (BER), de Réparation par Excision de Nucléotides (NER).

La concentration en extraits est de 0,2 mg/mL.

Chaque échantillon est testé sur 2 puces. Chaque puce comporte 4 spots par lésion. Les résultats sont ensuite normalisés (Normalizelt).

La valeur du plasmide Contrôle (pas de lésion) est soustraite à la valeur de l'intensité totale obtenue pour chaque lésion.

Les résultats sont donnés en Intensité de Fluorescence totale pour chaque lésion +/- Ecart-Type.

### 2. Résultats

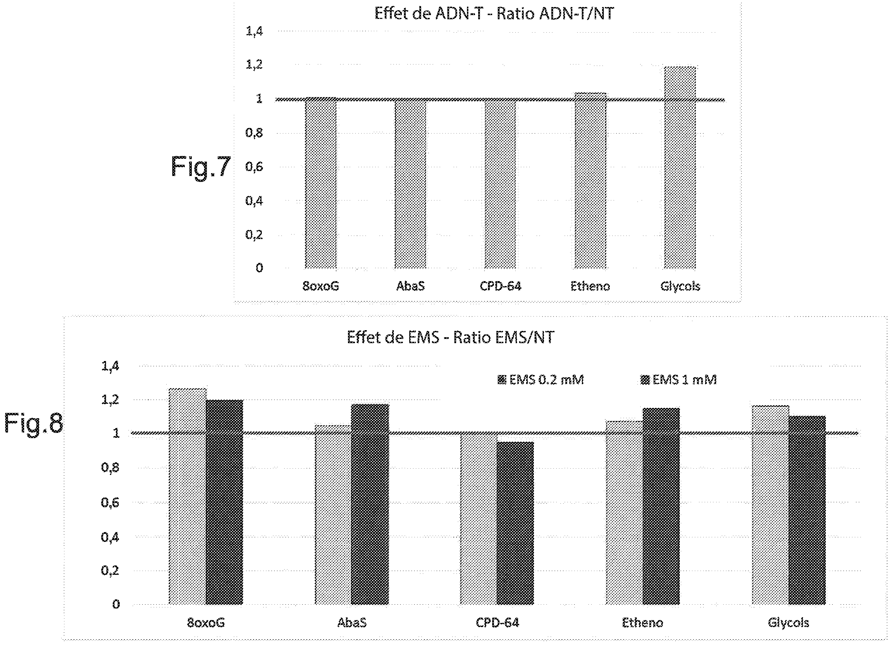

Les résultats sont présentés sur les Figures 7, 8 et 9.

Sur la Figure 7, on constate qu'à la dose non cytotoxique testée, le produit "ADN-Téloméractives" n'a aucun effet sur les systèmes de réparation de l'ADN, à l'exception d'un net effet simulateur de la réparation des glycols.

Sur la Figure 8, on constate qu'au moins à la plus forte concentration testée, l'EMS stimule exclusivement la Réparation par Excision de Base (BER). La réparation des photoproduits (CPD-64), pris en charge par la réparation par Excision de Nucléotides (NER) n'est pas affectée. Ce résultat est en faveur de la formation par l'EMS de petites lésions de types alkylations ou oxydations, ce qui induirait l'activation des enzymes les prenant en charge.

Ce résultat est conforme à ce qui est attendu.

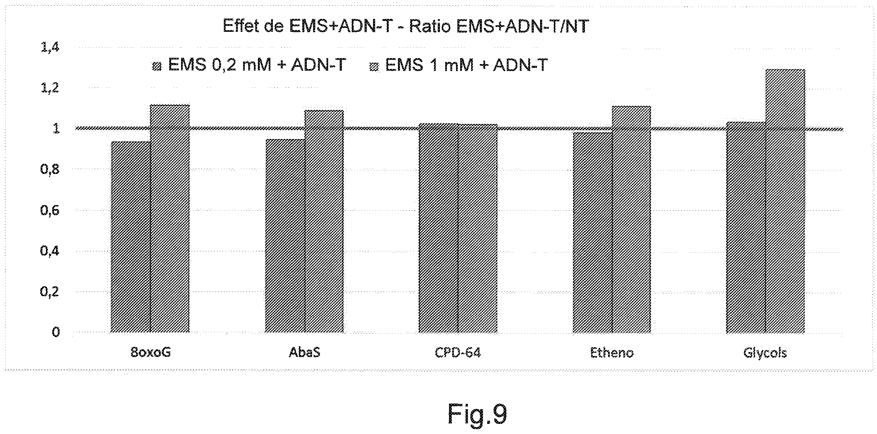

Enfin, sur la Figure 9, on constate que le prétraitement des cellules par le produit "ADN-Téloméractives" annule complètement l'impact d'EMS utilisé à la plus faible concentration, sur les systèmes de réparation de l'ADN.

En revanche, il persiste une induction des systèmes de réparation de l'ADN à la plus forte concentration d'EMS. Cette induction est cependant plus faible qu'avec EMS seul, sauf pour la lésion glycols. La stimulation de la réparation de la lésion Glycols évoque l'effet du produit "ADN-Téloméractives".

Par conséquent :
- Le produit "ADN-Téloméractives" seul, à dose non cytotoxique, a une action significative de stimulation de la réparation des glycols.
- Le prétraitement par le produit "ADN-Téloméractives" annule les effets de la plus faible dose d'EMS et réduit les effets de la plus forte dose.
- A la plus faible dose d'EMS, le produit "ADN-Téloméractives", en quantité suffisante, inactive l'EMS vis-à-vis de ses effets génotoxiques sur l'ADN.
- A la plus forte dose d'EMS, le produit "ADN-Téloméractives", probablement en quantité limitante, ne suffirait pas à inactiver la totalité de l'EMS présent, mais en réduirait l'impact.

### EXEMPLE 5 : Effet de certains principes actifs d'un extrait de Rhodiole et d'un extrait d'Astragale pour une utilisation selon l'invention

### 1. Matériel et méthodes

### 1.1 Produits

Les produits suivants ont été utilisés :
- Le premier extrait d'Astragale tel que décrit à l'Exemple 1 (obtenu par extraction hydroalcoolique de racines broyées *d'Astragalus membranaceus).Cet* extrait comporte au moins 4% en poids d'un mélange d'astragalosides I, II, III, IV, V, VI et VII, sur le poids total d'extrait sec d'Astragale, mais ne comporte pas de polysaccharides ;
- Le second extrait d'Astragale tel que décrit à l'Exemple 1 (obtenu par extraction à l'eau pure de racines broyées *d'Astragalus membranaceus*). Cet extrait comporte au moins 20% en poids de polysaccharides, sur le poids total d'extrait sec d'Astragale, mais ne comporte pas d'astragalosides ;

- L'extrait de Rhodiole tel que décrit à l'Exemple 1 (Extrait 1 de Rhodiole), comportant au moins 3% en poids de rosavine et au moins 1% en poids de salidroside, sur le poids total d'extrait sec de Rhodiole ;
- Un extrait de Rhodiole comportant au moins 3% en poids de salidroside (Extrait 2 de Rhodiole), sur le poids total d'extrait sec de Rhodiole, mais ne comportant pas de rosavine (fournit par Gonmisol) ;
- La combinaison d'extraits de Rhodiole et d'Astragale décrite à l'Exemple 1, ci-après désignée combinaison 4, l'extrait de Rhodiole comportant au moins 3% en poids de rosavine et au moins 1% en poids de salidroside, sur le poids total d'extrait sec de Rhodiole, et l'extrait d'Astragale comportant au moins 16% en poids de polysaccharides et au moins 0,8% en poids d'un mélange d'astragalosides I, II, III, IV, V, VI et VII, sur le poids total d'extrait sec d'Astragale.

### Préparation de combinaisons d'extraits

Outre la combinaison 4 dont la préparation est décrite à l'Exemple 1, trois autres combinaisons d'extraits sont préparées :
- Combinaison 1 : cette combinaison est préparée par mélange du second extrait d'Astragale (ne comportant pas d'astragalosides) et de l'extrait 1 de Rhodiole dans un ratio en poids de 4 : 5 ;
- Combinaison 2 : cette combinaison est préparée par mélange du premier extrait d'Astragale (ne comportant pas de polysaccharides) et de l'extrait 1 de Rhodiole dans un ratio en poids de 1 : 5 ;
- Combinaison 3 : cette combinaison est préparée par mélange du premier extrait Astragale, du second extrait d'Astragale et de l'extrait 2 de Rhodiole ne comportant pas de rosavine mentionné ci-dessus, dans un ratio en poids 3 : 12 : 5.

### Préparation de solutions aqueuses à partir des combinaisons d'extraits

Pour chaque combinaison, deux solutions aqueuses ont été fraîchement préparées afin de comparer les effets des astragalosides, des polysaccharides et de la rosavine, les concentrations étant indiquées dans les Tableaux 2 et 3 ci-après.

**Tableau 2 : Concentrations des solutions aqueuses testées pour l'essai A**

| Combinaison | Concentration combinaison (mg/mL) | Rosavine (mg/mL) | Salidroside (mg/mL) | Polysacharides (mg/mL) | Astragalosides (mg/mL) |
|---|---|---|---|---|---|
| 1 | 0,0225 | 0,0003750 | 0,0001250 | 0,0020000 | - |
| 2 | 0,0150 | 0,0003750 | 0,0001250 | - | 0,0001000 |
| 3 | 0,0167 | - | 0,0001250 | 0,0020000 | 0,0001000 |
| 4 | 0,0250 | 0,0003750 | 0,0001250 | 0,0020000 | 0,0001000 |

**Tableau 3 : Concentrations des solutions aqueuses testées pour l'essai B**

| Combinaison | Concentration combinaison (mg/mL) | Rosavine (mg/mL) | Salidroside (mg/mL) | Polysacharides (mg/mL) | Astragalosides (mg/mL) |
|---|---|---|---|---|---|
| 1 | 0,0450 | 0,0007500 | 0,0002500 | 0,0040000 | - |
| 2 | 0,0300 | 0,0007500 | 0,0002500 | - | 0,0002000 |
| 3 | 0,0333 | - | 0,0002500 | 0,0040000 | 0,0002000 |
| 4 | 0,0500 | 0,0007500 | 0,0002500 | 0,0040000 | 0,0002000 |

### 1.2 Culture cellulaire et différenciation

Des cellules SH-SY5Y ont été cultivées et repiquées selon les recommandations du fournisseur.

Tous les essais ont été réalisés dans trois réplicats techniques en utilisant des microplaques à 96 puits. Après l'ensemencement, les cellules SH-SY5Y ont été différenciées par exposition à la staurosporine (25 nM dans un milieu de culture complet à 1% de sérum de veau foetal) pendant 3 jours.

### 1.3 Fixation cellulaire et coloration

Après chaque essai, les cellules ont été fixées en utilisant un mélange de formaldéhyde et de méthanol (commercialisé par Sigma-Aldrich sous le nom « Formalin 10% » à température ambiante pendant 10 minutes, suivi d'une perméabilisation dans du Tween 20 à 0,5% (P2287 commercialisé par Sigma-Aldrich) pendant 30 minutes et enfin coloration des noyaux avec Hoechst et immunocoloration indirecte de la tubuline β3 pour distinguer les neurites (anticorps de lapin anti tubuline β3 (ab18207 commercialisé par Abcam) et anticorps secondaire anti-IgG de lapin conjugué à un fluorochrome Alexa 488 (commercialisé par Ozyme)) pendant 1h.

### 1.4 Test curatif

Les cellules ont été exposées pendant 2 jours avec 10 µM d'Aftin-5 seul. Ensuite, elles ont été traitées 48 heures avec les solutions aqueuses indiquées dans le

Tableau 2 ou simplement avec de l'eau (contrôle), en co-incubation avec de l'Aftin-5 (10 µM). Après la fixation et l'étiquetage, l'excroissance des neurites a été évaluée en utilisant MetaXpress (Molecular Devices).

### 1.5 Imagerie cellulaire et analyse d'images

Les images ont été obtenues à l'aide d'un microscope automatisé ImageXpress^{®} Micro Confocal System (Molecular Devices), avec un objectif 20X. Deux filtres dichroïques ont été utilisés simultanément pour détecter spécifiquement les différentes sondes utilisées. Quatre images par puits ont été enregistrés et analysés individuellement.

Les images ont été analysées en utilisant le logiciel MetaXpress (Molecular Devices).

### 2. Résultats sur l'allongement des neurites

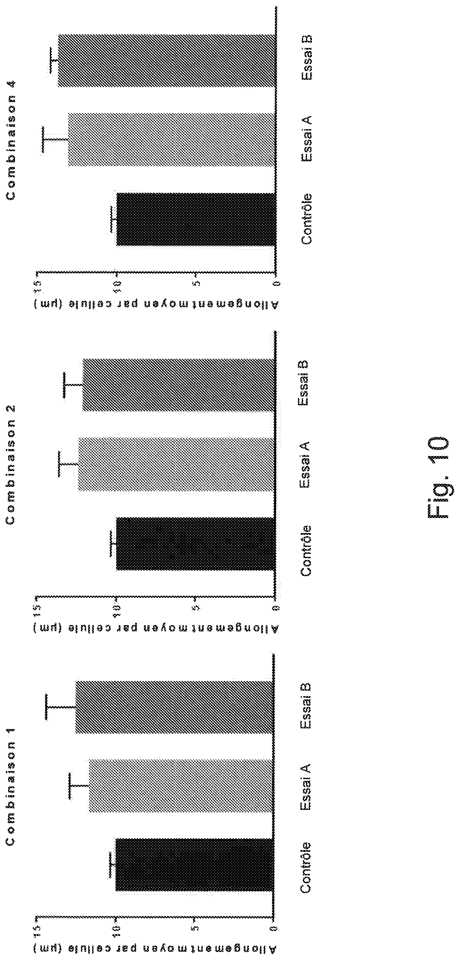

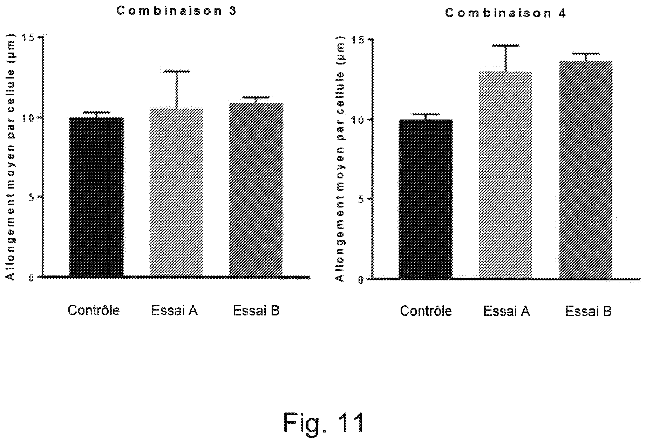

Les résultats concernant l'allongement des neurites sont présentés sur la Figure 10 pour les combinaisons 1, 2 et 4, et sur la Figure 11 pour les combinaisons 3 et 4.

### 2.1 Influence de la combinaison des astragalosides et des polysaccharides

Les résultats des essais A et B montrent que les combinaisons 1 et 2 permettent un allongement des neurites par rapport au contrôle. En effet, la combinaison 1 permet un allongement des neurites d'environ 16 % dans l'essai A et d'environ 26 % dans l'essai B, et la combinaison 2 permet un allongement des neurites d'environ 24 % dans l'essai A et d'environ 21 % dans l'essai B.

Toutefois, l'allongement des neurites obtenu dans le cas de la combinaison 1 (ne comportant pas d'astragalosides) ou de la combinaison 2 (ne comportant pas de polysaccharides) est inférieur en comparaison à l'allongement des neurites obtenu dans le cas de la combinaison 4. En effet, la combinaison 4 permet un allongement des neurites d'environ 32 % dans l'essai A et même d'environ 40 % dans l'essai B.

Par conséquent, une combinaison d'extraits comprenant à la fois des astragalosides et des polysaccharides permet d'obtenir un allongement des neurites supérieur à une combinaison d'extraits ne comprenant qu'un seul de ces principes actifs.

### 2.2 Influence de la rosavine

Les résultats des essais A et B montrent que la combinaison 3, ne comportant pas de rosavine, permet un allongement des neurites d'environ 5 % dans l'essai A et d'environ 8 % dans l'essai B, par rapport au contrôle.

Toutefois, l'allongement des neurites obtenu dans le cas de cette combinaison 3 est bien inférieur en comparaison à l'allongement des neurites obtenu dans le cas de la combinaison 4. En effet, la combinaison 4 permet un allongement des neurites d'environ 32 % dans l'essai A et même d'environ 40 % dans l'essai B.

Par conséquent, une combinaison d'extraits comprenant de la rosavine permet d'obtenir un allongement des neurites bien supérieur à une combinaison d'extraits n'en comprenant pas.

## Revendications

1. Produit contenant un extrait de Rhodiole et un extrait d'Astragale comme produits de combinaison pour une utilisation simultanée, séparée ou étalée dans le temps dans le traitement d'une maladie neurodégénérative, ledit extrait de Rhodiole étant un extrait de *Rhodiola rosea* et ledit extrait d'Astragale étant un extrait *d'Astragalus membranaceus* dans lequel l'extrait de *Rhodiola rosea* comporte au moins 1,5% en poids de rosavine sur le poids total d'extrait sec de *Rhodiolia rosea.*

2. Produit pour une utilisation selon la revendication 1, pour le traitement de la maladie d'Alzheimer.

3. Produit pour une utilisation selon la revendication 1, pour le traitement de la maladie de Parkinson.

4. Produit pour une utilisation selon l'une quelconque des revendications 1 à 3, pour une utilisation simultanée.

5. Produit pour une utilisation selon l'une quelconque des revendications 1 à 4, pour un traitement préventif et/ou curatif.

6. Produit pour une utilisation selon la revendication 5, pour un traitement curatif de neurones.

7. Produit pour une utilisation selon l'une quelconque des revendications 1 à 6, comportant une dose d'au moins 50 mg/jour d'un extrait de *Rhodiola rosea.*

8. Produit pour une utilisation selon l'une quelconque des revendications 1 à 7, dans lequel l'extrait de Rhodiola rosea comporte au moins 0,25% en poids de salidroside sur le poids total d'extrait sec de Rhodiola rosea.

9. Produit pour une utilisation selon l'une quelconque des revendications 1 à 8, comportant au moins une dose de 50 mg/jour d'un extrait *d'Astragalus membranaceus.*

10. Produit pour une utilisation selon l'une quelconque des revendications 1 à 9, dans lequel l'extrait *d'Astragalus membranaceus* comporte au moins 0,2% en poids d'astragalosides sur le poids total d'extrait sec *d'Astragalus membranaceus.*

11. Produit pour une utilisation selon l'une quelconque des revendications 1 à 10, dans lequel l'extrait *d'Astragalus membranaceus* comporte au moins 10% en poids de polysaccharides sur le poids total d'extrait sec *d'Astragalus membranaceus.*

12. Produit pour une utilisation selon l'une quelconque des revendications 1 à 11, dans lequel le ratio en poids sec de l'extrait d'Astragale sur l'extrait de Rhodiole est compris entre 0,5 et 1,5.

13. Produit pour une utilisation selon l'une quelconque des revendications 1 à 12, comportant en outre un ingrédient choisi parmi le groupe constitué par le tocophérol, le sélénium, le silicium, le zinc, le magnésium, les polyphénols autres que ceux issus des extraits de Rhodiole et d'Astragale, ou leurs mélanges.

## Patentansprüche

1. Produkt, das einen Rhodiola-Extrakt und einen Astragalus-Extrakt als Kombinationsprodukte für eine gleichzeitige, getrennte oder zeitlich gestaffelte Verwendung in der Behandlung einer neurodegenerativen Erkrankung enthält, wobei der Rhodiola-Extrakt ein *Rhodiola* rosea-Extrakt ist und der Astragalus-Extrakt ein *Astragalus membranaceus*-Extrakt ist, wobei der *Rhodiola* rosea-Extrakt, auf das Gesamtgewicht des *Rhodiola* rosea-Trockenextrakts bezogen, mindestens 1,5 Gewichts-% Rosavin umfasst.

2. Produkt für eine Verwendung nach Anspruch 1 zur Behandlung der Alzheimer-Erkrankung.

3. Produkt für eine Verwendung nach Anspruch 1 zur Behandlung der Parkinson-Erkrankung.

4. Produkt für eine Verwendung nach einem der Ansprüche 1 bis 3 für eine gleichzeitige Verwendung.

5. Produkt für eine Verwendung nach einem der Ansprüche 1 bis 4 für eine vorbeugende und/oder heilende Behandlung.

6. Produkt für eine Verwendung nach Anspruch 5 für eine heilende Behandlung von Neuronen.

7. Produkt für eine Verwendung nach einem der Ansprüche 1 bis 6, das eine Dosis von mindestens 50 mg/Tag eines *Rhodiola* rosea-Extrakts umfasst.

8. Produkt für eine Verwendung nach einem der Ansprüche 1 bis 7, wobei der *Rhodiola* rosea-Extrakt, auf das Gesamtgewicht des *Rhodiola* rosea-Trockenextrakts bezogen, mindestens 0,25 Gewichts-% Salidrosid umfasst.

9. Produkt für eine Verwendung nach einem der Ansprüche 1 bis 8, das mindestens eine Dosis von 50 mg/Tag eines *Astragalus membranaceus*-Extrakts umfasst.

10. Produkt für eine Verwendung nach einem der Ansprüche 1 bis 9, wobei der *Astragalus membranaceus-*Extrakt, auf das Gesamtgewicht des *Astragalus membranaceus-*Trockenextrakts bezogen, mindestens 0,2 Gewichts-% Astragaloside umfasst.

11. Produkt für eine Verwendung nach einem der Ansprüche 1 bis 10, wobei der *Astragalus membranaceus-*Extrakt, auf das Gesamtgewicht des *Astragalus membranaceus-*Trockenextrakts bezogen, mindestens 10 Gewichts-% Polysaccharide umfasst.

12. Produkt für eine Verwendung nach einem der Ansprüche 1 bis 11, wobei das Trockengewichtsverhältnis des Astragalus-Extrakts zum Rhodiola-Extrakt im Bereich zwischen 0,5 und 1,5 liegt.

13. Produkt für eine Verwendung nach einem der Ansprüche 1 bis 12, das weiter einen Inhaltsstoff umfasst, ausgewählt aus der Gruppe bestehend aus Tocopherol, Selen, Silicium, Zink, Magnesium, Polyphenolen, die nicht aus dem Rhodiola- und dem Astragalus-Extrakt stammen, oder deren Mischungen.

## Claims

1. Product containing an extract of Rhodiola and an extract of Astragalus as combination products for a simultaneous, separate or spread over time use in the treatment of a neurodegenerative disease, said extract of Rhodiola being an extract of *Rhodiola rosea* and said extract of Astragalus being an extract of *Astragalus membranaceus* wherein the extract of *Rhodiola rosea* comprises at least 1.5% by dry weight of rosavin with respect to the total weight of dried extract of *Rhodiola rosea.*

2. Product for use according to claim 1, for the treatment of Alzheimer's disease.

3. Product for use according to claim 1, for the treatment of Parkinson's disease.

4. Product for use according to any one of claims 1 to 3, for a simultaneous use.

5. Product for use according to any one of claims 1 to 4, for a preventive and/or curative treatment.

6. Product for use according to claim 5, for a curative neuron treatment.

7. Product for use according to any one of claims 1 to 6, comprising a dose of at least 50 mg/day of an extract of *Rhodiola rosea.*

8. Product for use according to any one of claims 1 to 7, wherein the extract of Rhodiola rosea comprises at least 0.25% by dry weight of salidroside with respect to the total weight of dried extract of *Rhodiola rosea.*

9. Product for use according to any one of claims 1 to 8, comprising at least one dose of 50 mg/day of an extract of *Astragalus membranaceus.*

10. Product for use according to any one of claims 1 to 9, wherein the extract of *Astragalus membranaceus* comprises at least 0.2% by weight of astragalosides with respect to the total weight of dried extract of *Astragalus membranaceus.*

11. Product for use according to any one of claims 1 to 10, wherein the extract of *Astragalus membranaceus* comprises at least 10% by weight of polysaccharides with respect to the total weight of dried extract of *Astragalus membranaceus.*

12. Product for use according to any one of claims 1 to 11, wherein the ratio by dry weight of the extract of Astragalus with respect to the extract of Rhodiola is between 0.5 and 1.5.

13. Product for use according to any one of claims 1 to 12, further comprising an ingredient chosen from the group consisting of tocopherol, selenium, silicon, zinc, magnesium, polyphenols other than those derived from the extracts of Rhodiola and Astragalus, or mixtures thereof.
